# EUROPEAN PATENT SPECIFICATION

(11) **EP 2 349 001 B1**
(45) Date of publication and mention of the grant of the patent: **15.08.2012**
(21) Application number: 09778792.3
(22) Date of filing: 01.10.2009
(51) Int. Cl.: A61B 5/155, A61B 5/145, A61B 5/157

(54) **INSTRUMENT AND SYSTEM FOR PRODUCING A SAMPLE OF A BODY LIQUID AND FOR ANALYSIS THEREOF**
INSTRUMENT UND SYSTEM ZUR ERZEUGUNG EINER PROBE EINER KÖRPERFLÜSSIGKEIT UND ZU DEREN ANALYSE
INSTRUMENT ET SYSTEME POUR PRODUIRE UN ECHANTILLON DE FLUIDE CORPOREL POUR ANALYSE

(30) Priority: 29.10.2008 EP 08018873
(43) Date of publication of application: 03.08.2011
(73) Proprietor: Roche Diagnostics GmbH, 68305 Mannheim (DE); F. Hoffmann-La Roche AG, 4070 Basel (CH)
(72) Inventor: HOERAUF, Christian, 68723 Oftersheim (DE)
(74) Representative: Durm & Partner
(86) International application number: PCT/EP2009/007042
(87) International publication number: WO 2010/049048

(56) References cited:
- EP-A- 1 360 933
- EP-A- 1 362 551
- EP-A- 1 527 737
- WO-A-2006/058654
- WO-A-2008/022755
- US-A1- 2007 016 103
- US-B2- 6 749 618

## Description

The invention relates to the collection of samples of body liquid for allowing analysis thereof, i.e. determination of an analyte concentration therein. In particular it relates to instruments and systems for producing a small sample of body liquid by piercing the skin of a subject (human or animal) using a disposable lancing element having a skin piercing tip suitable for generating a small wound from which the sample is drawn. Depending on the skin site used and on the lancing depth the body liquid is blood or interstitial liquid or a mixture thereof.

Analysis based on skin-piercing is important in several fields of medical diagnostics and treatment. Of particular importance is the field of diabetes management. It has been determined that severe long term damages caused by diabetes mellitus can be avoided if the patient controls her or his blood sugar level several times a day in order to adapt the required insulin injections closely to the actual need for maintaining a constant blood sugar level. This requires so called "home-monitoring" by the patient himself or by other people not having a medical training.

Other important fields of medical diagnostics and treatment with similar requirements, including home-monitoring, refer for example to the regular control of blood cholesterol and to the control of blood coagulation parameters. The invention is in particular suitable but not limited to home-monitoring applications. Similar requirements also exist, e.g., in so called "near-patient-testing".

Lancing of the skin is generally performed by a lancing system comprising, as mutually adapted components of the system, a reusable hand-held instrument and lancing elements. The movement required for lancing (puncturing movement) is driven by a lancing drive provided inside a housing of the instrument and adapted for driving a lancing element connected thereto. Lancets can be interchangeably connected to the drive and generally are disposible items.

After triggering the puncturing movement the lancet is driven in a puncture direction until it reaches a point of maximum displacement and thereafter it is further driven in a reverse direction. Many suitable lancet drive mechanisms have been described. In most cases the driving force is supplied by a tensioned spring and the lancet drive further includes suitable mechanical means for converting the force of the spring into the required movement of a lancet.

An important consideration in developing lancing systems is the pain caused by the pricking action. This pain and the convenience of use are decisive factors determining compliance of the patient, i.e. his willingness to perform regular analyses as required for maintaining his health. It has been determined that reliable production of the required amount of sample liquid with minimum pain highly depends on the reproducibility of an optimum penetration depth of the tip of the lancing element into the skin (see US-patent 5,318,584).

With earlier lancet systems the analysis generally required a plurality of steps to be performed by the user. After lancing with such earlier systems the blood did not readily emanate from the wound site in the lanced skin. Therefore manual "milking" steps such as pinching, squeezing and kneading where necessary in order to express the required amount of sample liquid. Finally sample liquid was contacted to an analysis element of an analysis system (which was separate and distinct from the lancing system) and the analysis was performed thereby.

In order to improve the production of sample liquid at the lancing site and to avoid the manual "milking" several proposals were made all of which relate to the design of the contact area at a distal end of the lancing instrument having a (generally ring-shaped) skin contact surface surrounding a skin contact opening. Such lancing systems are described in
WO 99/26539
WO 01/89383 A2
EP 1 245 187 A1
EP 1 586 269
EP 1 586 270

While these approaches differ in several ways, a common feature thereof is that the skin contact opening has a relatively large diameter whereby the skin bulges into the skin contact opening forming a target site bulge which penetrates to some extent into the opening when the lancing instrument is pressed with its distal end (i.e. with the skin contact surface) against the skin. This bulging action (hereafter designated "target site bulging") is generally combined with additional means for improving sample liquid production, such as a mechanical squeezing acting radially inwardly, a pumping action involving axial movement of parts of the instrument, etc.

Ideally these measures allow with a high success rate (preferably better than 90 %) expression of a sufficient amount of sample liquid without manual "milking". This again is a requirement of integrated lancing and analysis systems which, in a single instrument, comprise both, means for the lancing-type sample production and means for the analysis. Such integrated systems are designed to perform the sample generation and analysis fully automatically, i.e. without any handling steps by the user.

They have been proposed in a plurality of variants which can be assigned to two types, namely
A) "Two unit systems" having - in a single instrument housing - two separate units for lancing and for analysis. Typically the units are moved one after the other to a common skin contact opening (see e.g. EP 1 669 028 A1 and EP 1 736 100 A1)
B) "Single unit systems" operating with a single combined lancing- and analysis unit suitable for performing both functions (lancing and analysis). Most such systems operate with integrated lancing and analysis elements. The two components of such combined lancing and analysis elements are generally manufactured separately but assembled by the manufacturer or at least before use, i.e. before the lancing movement is triggered. In the instrument such elements are processed as a unified item. In other single unit systems both functions (lancing and analysis) are performed by the same unit but a lancing element and an analysis element are provided and processed separately during at least a part of the analytical procedure. Examples of single unit systems are described in the following publications:
   WO 01/72220
   WO 03/009759 A1
   EP 1 342 448 A1
   EP 1 360 933 A1
   EP 1 362 551 A1

Even though several of the discussed systems, in particular integrated lancing and analysis systems, provide improved results as compared to earlier known devices, there are still substantial shortcomings. There is a need for improvements with respect to several partially contradictory requirements including ease of use, minimum pain and minimum requirements concerning volume, weight and production cost.

With a view to this aim a first aspect of the invention proposes an instrument and a system for producing a sample of body liquid by piercing the skin comprising
- a housing
- a lancing drive within said housing adapted for being connected to a lancing element and adapted for driving a lancing element connected thereto in a puncturing movement in which the lancing element moves, after triggering the puncturing movement, in a puncture direction until it reaches a point of maximum displacement and in a reverse direction after it has reached the point of maximum displacement,
- a pressure ring surrounding a skin contact opening and being adapted for being pressed against the skin,
wherein the skin contact opening has an opening area corresponding to a circle with a diameter of at least 1.5 mm and not more than 4 mm. Preferably the opening area corresponds to a circle with a diameter of at least 2 mm, more preferably at least 2.5 mm. The instrument .further comprises a pressing force control device for controlling the pressing force between the pressure ring and the skin at the time of triggering the puncturing movement, to be at least 3 N, preferably at least 4 N and most preferably at least 5 N.

Generally the pressure ring is circular and in this case it should have the specified inner (free) diameter. In case of a non-circular skin contact opening the area of the opening should correspond (i.e. be the same) as the area of a circle with the mentioned diameter values.

In contrast to the above mentioned known approaches the invention does not rely on target site bulging for automatic expression of a sufficient amount of sample liquid. Rather the inventors have found that it is possible to achieve the required high reliability of sample generation with a combination of a relatively small skin contact opening and a relatively high pressure force which is guaranteed by a pressing force control device. This was unexpected, in particular because it had been assumed that such a high pressure would "press away" the blood, unless the device has a large opening allowing substantial bulging of the skin. This assumption was based - inter alia - on the experience known even to a layperson that pressing with a thin item onto the skin causes a color change from red to white indicating a loss of blood from the respective site.

The invention overcomes a problem related to target site bulging namely the lack of reproducibility of the penetration depth by which the tip of the lancing element penetrates into the skin. With a given adjustment of the longitudinal position (i.e. position in the direction of the lancing movement; hereafter "z-position") of the lancing drive and consequently a given z-position of the point of maximum displacement of the lancet the penetration depth depends on the exact z-position of the skin surface during the puncturing movement. Due to the bulging this skin position is substantially undefined.

In order to overcome this uncertainty some prior art devices have means for detection of the exact z-position of the skin by a skin position detection device integrated into the lancing instrument. Others operate with a penetration depth reference element having a reference skin contact surface which is contacted to the skin (additionally to the skin contact surface surrounding the skin contact opening of the instrument), for providing a reliable z-position reference during penetration of the lancing element tip into the skin. While these approaches help to achieve a reproducible penetration depth they require a substantial expense in the instrument design and production, making the system less handy and more costly.

Many lancing systems simply disregard the penetration depth uncertainty. While individual variations from person to person may be tolerable because they can be compensated by adjusting the penetration depth setting, intra-individual variations (i.e. variations referring to the same user) remain. These include changes of the (elastic and other) properties of the skin caused by influencing factors such as temperature, previous skin treatment (e.g. washing with soap) and choice of the particular lancing site. These variations lead to a much larger pain than necessary, because they require a high value of the penetration depth setting to make sure that a sufficient amount of sample liquid is produced even with a disadvantageous position of the skin.

The invention allows an excellent reproducibility of the z-position of the skin at the lancing site and thus an excellent reproducibility of the penetration depth. Simultaneously automatic sample generation (without "milking") is achieved with a simple and inexpensive design of the lancing system.

A defined pressing force is ensured by a suitable pressing force control device. Such a device can be mechanical, in particular comprising a spring device which is arranged in such a manner that its spring force acts between the pressure ring and the housing. The spring device is preferably embodied as a metal spring. Other spring-like devices are, however, known and can be used, such as a pneumatic spring or a resilient element of an elastic material. Hereafter the term "spring" is used as an example of any such spring device. Preferably it is pre-tensioned, as will be described in more detail below.

Pressing force control devices operating by electrical means may comprise an electromagnetic drive including a coil and a magnetic core, in particular a voice coil drive. The control of the pressure can be fully automatic or it can require an activity of the user. In the latter case electrical means can be used to measure the force by which the pressure ring is pressed against the skin and this force can be indicated to the user by suitable visible, acoustic or tactile means, whereby the user can adapt the pressing force to the desired value.

In a preferred further aspect of the invention the pressure ring has on at least a part of its circumference a width of not more than 1.5 mm, preferably not more than 1.2 mm and most preferably not more than 1 mm. According to a highly preferred embodiment such a narrow pressure ring is surrounded by a peripheral surface portion which is recessed from the pressure ring such that the pressure ring protrudes in the puncturing direction from the peripheral surface portion. The pressure ring and the recessed peripheral surface portion may be embodied as part of a single element, designated pressure piece. However, it is also possible that the pressure ring and the recessed peripheral surface are parts of two separate elements.

According to a further preferred embodiment the pressure ring protrudes from the peripheral surface by at least 0.5 mm, preferably at least 0.8 mm and not more than 2.0 mm, preferably not more than 1.5 mm.

In the context of this main aspect it is of particular importance to note that the term "pressure ring" is used herein in an essentially geometrical sense, namely referring to a narrow ring-shaped area surrounding the skin contact opening. Forces acting between the instrument and the skin are borne essentially only, or at least preferentially, via the pressure ring. Thus the pressure ring is a narrow ring shaped surface area surrounding the skin contact opening and bearing during practical use of the instrument at least 50 %, preferably at least 70 % and most preferably at least 90 % of the forces acting between the instrument and the skin. Consequently according to this aspect of the invention at least the mentioned percentages of the total force acting between the skin and the instrument is borne only by a small ring shaped area of the described maximum dimensions.

The ring shaped area forming the pressure ring may be part of an annular element with circular cross-section, oftentimes designated as "ring". Preferably, however, the pressure ring is embodied as part of a pressure piece, as will be explained further with reference to the drawings.

In the context of the invention is has been found that the features of the further preferred aspect of the invention help to reliably and automatically generate a sufficient amount of sample liquid for analysis.

In a further preferred aspect of the invention the lancing drive and the pressure ring are so positioned relative to each other that a projecting distance by which the skin piercing tip of the lancing element extends out of a pressure ring plane, when the lancing element is in the point of maximum displacement, is not more than 0.3 mm, preferably not more than 0.2 mm and most preferably not more than 0.1 mm. The pressure ring plane is defined as a plane running perpendicular to the puncturing direction through the forwardmost part of the pressure ring. Thus the projecting distance is the distance in z-direction between the point of maximum displacement and the closest point (in z-direction) of the pressure ring.

In the context of the invention it has been found that surprisingly even with such extremely small projecting distances a highly reliable sample generation is achieved. In this context it is important to understand the difference between the reliability of sample generation and the amount of sample generation. It was unexpected that these two properties show little correlation. Extremely small projecting distances according to the third aspect of the invention may only generate small amounts of sample but still this occurs with a reliability which is sufficient for a fully automatic lancing and analysis device.

The further preferred aspects of the invention are favorable for any size of skin contact opening and for any pressing force. Particular good results are, however, achieved if the instrument comprises a pressing force control device for controlling the pressing force within the limits specified above with respect to the first main aspect of the invention. Furthermore the skin contact opening should preferably have an opening area corresponding to a circle with a diameter of at least 1.5 mm, preferably at least 2 mm, most preferably at least 2.5 mm and not more than 8 mm, preferably not more than 5 mm and most preferably not more than 3.5 mm.

The instrument is preferably adapted for operation with an integral lancing and analysis element, both forming a "single unit system" as specified above. Further preferentially in this case the lancet of the integral lancing and analysis element is embodied as a direct sampler having a capillary channel for transporting sample liquid produced by piercing the skin by means of capillary flow to a sample collection zone thereof.

Also it is preferred that a minimum interaction time period required for lancing and sampling a sufficient amount of sample liquid for analysis is at most 3 seconds. Preferably the minimum interaction time period is no more than 2 seconds and more preferably it is no more than 1 second.

This relates specifically to integrated lancing and analysis systems of both types A and B identified above. In such systems the user interacts with the system by establishing a pressing force between the skin and the pressure ring of the instrument. This can conveniently be done by pressing a hand-held instrument against the finger or other body part. Alternatively the finger or other body part can be pressed against an instrument, lying e.g. on a table.

According to the prior art, timing has generally been only a concern with respect to the "test time", i.e. the total time required for the analysis (from lancing until the analyte concentration is indicated). The inventors have found that - deviating from earlier understanding - the duration of the minimum interaction time period ("MITP") is highly critical for achieving the partially contradictory requirements mentioned above. This time period is defined as the minimum time duration for which user-instrument interaction (as specified above) is required for lancing and for collecting a sufficient amount of sample for the analysis in a sample collection device of the system. The functions performed during the MITP include lancing, expression of sample liquid from the tissue (preferably directly into a capillary of the lancing element) and collecting a sufficient amount of sample.

The MITP is a system-related quantity which is user-independent, i.e. only determined by the design of the instrument, and possibly by other components of the system. It must not be confused with the actual time of interaction which in each case depends on numerous aspects including the habits of the user. The actual interaction time generally varies between users and, even for a specific user, from analysis to analysis. The invention teaches to design the system in such a manner, that the minimum time for which at least every user must interact with the instrument, is below the indicated very small threshold values.

The starting point of the MITP is a point in time at which the system is "ready for lancing", i.e. the lancing drive is ready for driving a lancing movement of a lancing element connected thereto and the desired lancing site of the skin is properly located at the skin contact opening of the instrument. Depending on the design of the system a short period of time may be required between establishing the status "ready for lancing" and the triggering of the puncturing movement. Such a short (preparatory) delay period may be required by the instrument, for example for detecting the skin position. Preferably, however, the design is such that no such preparatory time period is needed due to instrumental requirements, i.e. the triggering can immediately take place when the status of the system is "ready for lancing". In this case the starting point of the MITP may coincide with the triggering of the puncturing movement.

A very short and well defined preparatory delay period may, however, be provided for non-instrumental reasons, in particular to take into account visco-elastic deformation of the skin which takes place after establishing a pressure force between the skin and the pressure ring.

The end of the MITP is marked by the fact that a sufficient amount of body liquid has been sampled, i.e. is available in the sample collection device of the instrument for analysis. A "sample collection device" as used herein is any part of the system, inside the instrument, in which sample liquid produced as a result of skin lancing is available for analysis. It can, for example, be a chamber or capillary and can be empty, or filled with bibulous material.

Details depend on the type and design features of the particular system.
- In the case of a "two unit system" the sample collection device belongs to the analysis unit. It can be a part of an analysis element or of a dedicated sample collection element and collects sample after the analysis unit has been moved to the skin contact opening.
- In the case of a "single unit system" the sample collection device can be a part of a lance element, a part of an analysis element, a part of an integrated lancing and analysis element or a part of a dedicated sample collection element.
- If the sample collection device is a part of an analysis element or of an integrated lancing and analysis element it can, in particular, be a part of a reaction zone thereof containing reagents which react with the sample liquid, thereby producing some kind of measurable physical change which is characteristic for the analysis.
- Preferably the sample collection device is separate from the reaction zone of the analysis element and includes a reservoir, which is suitable for storing a sample liquid for an intermediate storage time which is longer than the MITP. One advantage of this embodiment is that it allows to separate the timing requirements of the sample collection from the timing requirements of the analysis. The MITP is terminated as soon as the reservoir of the sample collection device contains a sufficient amount of sample liquid for the analysis. Further steps, including for example the filling of a reaction zone, can take place separately without continued interaction of the user.
- In the last mentioned preferred embodiment the transfer of the sample liquid from the reservoir of the sample collection device to the reaction zone of the analysis element can either take place spontaneously or with controlled timing. In the former case permanent fluid communication is provided between the reservoir and the reaction zone. In the latter case the fluid communication from the reservoir of the sample collection device to the analysis element is "switchable", i.e. initially, preferably at least for the duration of the MITP, there is no fluid communication but same is established in a controlled manner at a suitable point of time. Suitable means for such switching are known for example from WO 2005/084546.

The value of the pressing force during the entire MITP is important. It should preferably be maintained by means of the pressing force control device to be at least 3 N, preferably at least 4 N and more preferably at least 5 N during the MITP. According to another preferred embodiment maximum values should be maintained during the same period, namely at most 10 N, preferably at most 8 N and more preferably at most 7 N.

These limiting values of the pressure force are preferred with respect to the requirements of withdrawing sample from the skin. However, this does not mean that the pressing force should be allowed to float in that range during the MITP. Rather it has been found that the maximum variation range of the pressing force should be limited to no more than 15 %, preferably no more than 10 % and more preferably to no more than 5 %. Expressed in absolute values the maximum variation range of the pressing force between the pressure ring and the skin during the MITP should be no more than +/- 0.5 N, preferably not more than +/- 0.3 N and more preferably not more than +/- 0.2 N.

As noted above the MITP is a user-independent quantity which depends only on the design of the system. Preferably, however, the instrument comprises a MITP controlling device. This term refers to any device which helps to make sure that the required interaction between the user and the instrument (i.e. mainly the required pressing force between the skin and the pressure ring) is maintained by the user at least during the MITP. In other words the MITP controlling device provides assistance to make sure that the actual interaction between the user and the instrument overlaps (or at least coincides with) the MITP.

The MITP controlling device need not operate fully automatically in the sense that no acts of the user, such as manual triggering of the puncturing movement, are required. Rather it may provide assistance to the user, in particular by signaling to the user directly or indirectly the start and the end of the MITP.

The MITP controlling device comprises means for detecting the starting point of the MITP, by detecting the pressing force acting between a pressure ring and the skin using any suitable means (to be discussed in more detail below). When the pressure corresponds to a predetermined minimum value or range this status can be indicated to the user by a suitable visible, audible or tactile signal. Alternatively the lancing movement can be triggered automatically when the status "ready for lancing" has been detected. In this case there may be no delay between "ready to lance" and triggering, i.e. the MITP starts with the automatic triggering. Alternatively there may be an instrument-controlled delay time, e. g. to take into account the time needed for visco-elastic skin-deformation. In such cases the preparatory delay period between "ready to lance" and triggering is preferably at most 1 sec, more preferably at most 0.7 sec and most preferably at most 0.5 sec. A preferred lower limit is 0.2 sec, at least 0.3 sec being more preferred and at least 0.4 sec being most preferred.

The end of the MITP period is generally indicated to the user by a suitable visible, audible or tactile signal.

It should be noted, that a dedicated MITP controlling device is not necessary for the invention in its most general sense. Depending on the particular situation it may be sufficient for the user to be provided with an indirect indication of the start and end of the MITP. For example the "ready to lance"-status can be "felt" by the user when pressing his finger on a spring-supported pressure ring (to be described in more detail below) and the duration of the MITP may be so short, that it may by sufficient to rely on the "feeling" of the user with respect to the end of the MITP.

The instrument may have some kind of fill control (as part of the MITP controlling device) indicating a sufficient amount of sample liquid or allowing an analysis only if a sufficient amount of sample has been collected. However, in many cases such fill control is not required. Rather the end of the MITP is calculated by the instrument using a fixed MITP value (depending on the design of the system components).

It has been found that substantial advantages are achieved with integrated lancing and analysis systems if the described pressing force between the pressure ring at the distal end of the lancing instrument and the skin is maintained not only at the time of lancing but also for a short interaction period thereafter:
- With both types A and B of integrated lancing and analysis systems, maintaining this pressing force for a MITP helps to produce a sufficiently large volume of sample liquid.
- In the case of type A (two unit systems) maintaining a MITP with the described pressing force is furthermore important to make sure that the position of the instrument (i.e. its skin contact opening) relative to the skin is fixed until the point of time that the analysis device is moved to the skin contact opening.
- With systems of type B (single unit systems) it has been found that maintaining a MITP with the described pressing force is important in order to allow a precise z-position of the lancing tip, thereby improving suctioning of a sufficient amount of sample liquid during a short period of time.

Furthermore it has been found that many users of integrated lancing and analysis systems have problems to maintain a sufficient pressing force for a sufficient period of time and that compliance with recommended rules of using the instrument is much better with a system incorporating the features described here.

The invention allows to use a simple and inexpensive design of penetration depth adjustment device. For example in order to adapt for small remaining variations of the skin position it may be sufficient to provide interchangeable distance elements or pressure rings to allow a single adaption of the systems to the needs of a particular user.

The instrument and system of the invention take into account the viscoelastic properties of the skin in an optimized manner. In this way not only a sufficient supply of sample liquid is ensured but also "flooding" by too much sample liquid is avoided. The invention allows reliable analysis preferably with very small sample volumes in the order not more than 300 nl, preferably not more than 200 nl, and most preferably not more than 100 nl.

The invention is hereafter described in more detail with reference to preferred embodiments shown in the drawings. The technical features and elements shown therein can be used individually or in combination to design embodiments of the invention. In the drawings
- Fig. 1: shows a schematic sketch of the skin pressed against a pressure ring;
- Fig. 2: shows a perspective view of an analysis instrument;
- Fig. 3: shows a longitudinal section of the instrument shown in Figure 2;
- Fig. 4: shows a perspective view of a lancing element for use in the instrument shown in Figure 3;
- Fig. 5: shows a schematic sketch regarding an aspect of the function of the instrument shown in Figure 3.

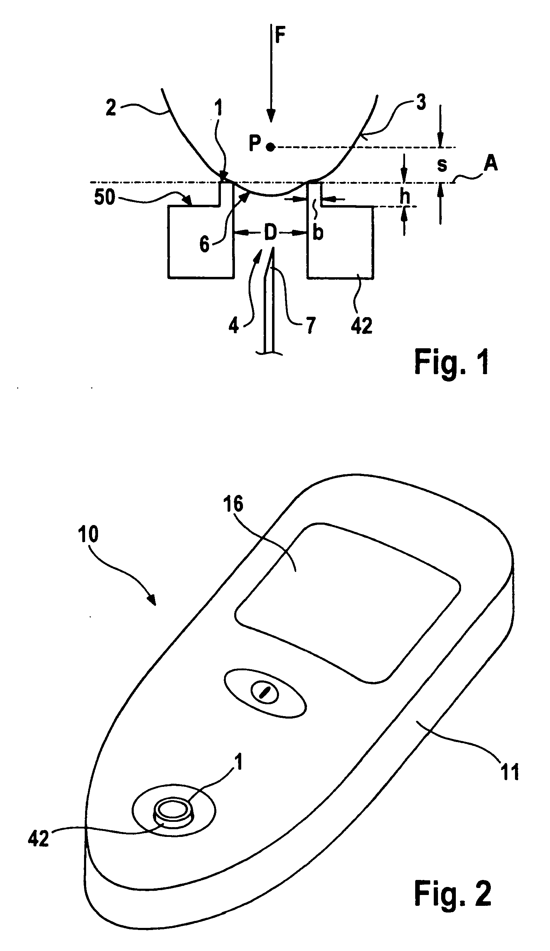

Figure 1 shows a view of a pressure ring 1 against which a finger tip 2 is pressed with force F. The skin 3 bulges into the skin contact opening 4 defined by pressure ring 1. The degree of this bulging, i.e. the distance between the plane A of pressure ring 1 and the apex of target site bulge 6 depends on the pressing force F, the dimensions of the skin contact opening 4 and the elastic properties of the skin which vary from person to person but also show intra-individual variation for consecutive uses by the same person.

Figure 1 also shows symbolically the point P of maximum displacement which a piercing tip 7 reaches on its movement path during the puncturing movement. With most lancing instruments the z-positions of the plane of the pressure ring 1 and of the point of maximum displacement P relative to each other (i.e. projecting distance s shown in Figure 1) can be adjusted in order to allow a penetration depth setting. Figure 1 shows that for a given value of projecting distances s the actual penetration depth depends directly on the degree of target site bulging.

Preferably the diameter D of the skin contact opening 4 is rather small. In this case there is only minimal target site bulging, whereby the reproducibility of the penetration depth of consecutive lancing events is excellent. Generation of a sufficient amount of sample liquid is supported by using a narrow pressure ring having a width b of not more than 1.5 mm. In the preferred embodiment shown the pressure ring 1 is a part of a pressure piece 42 which comprises a peripheral surface portion 50 surrounding the pressure ring 1 and recessed from the pressure ring 1. Thus, the pressure ring 1 protrudes by a protruding distance h from the peripheral surface portion 50.

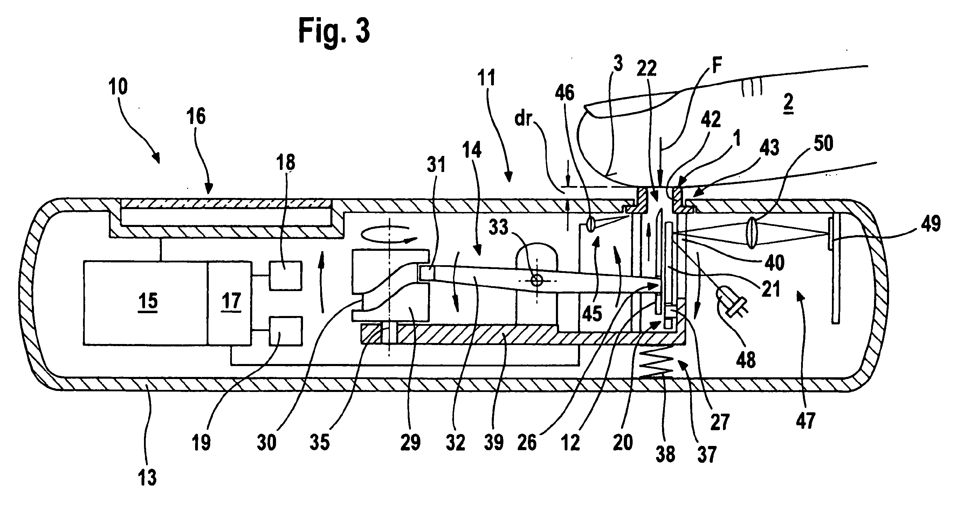

Figures 2 to 5 show a suitable lancing system 10. It includes a reusable hand-held instrument 11 and a disposable lancing element 12 with a piercing tip 7. A housing 13 of the instrument contains a lancing drive 14 and a measurement and evaluation electronics 15 shown only symbolically as a block in Figure 3. A display 16 is provided in order to allow visual indication of information (including status information concerning the system, advice concerning its handling, analytical results etc.) to the user. Optionally the instrument also comprises a MITP controlling device 17, a device 18 for generating audible signals (such as a buzzer) and/or a device 19 for generating tactile signals (such as a vibration generator).

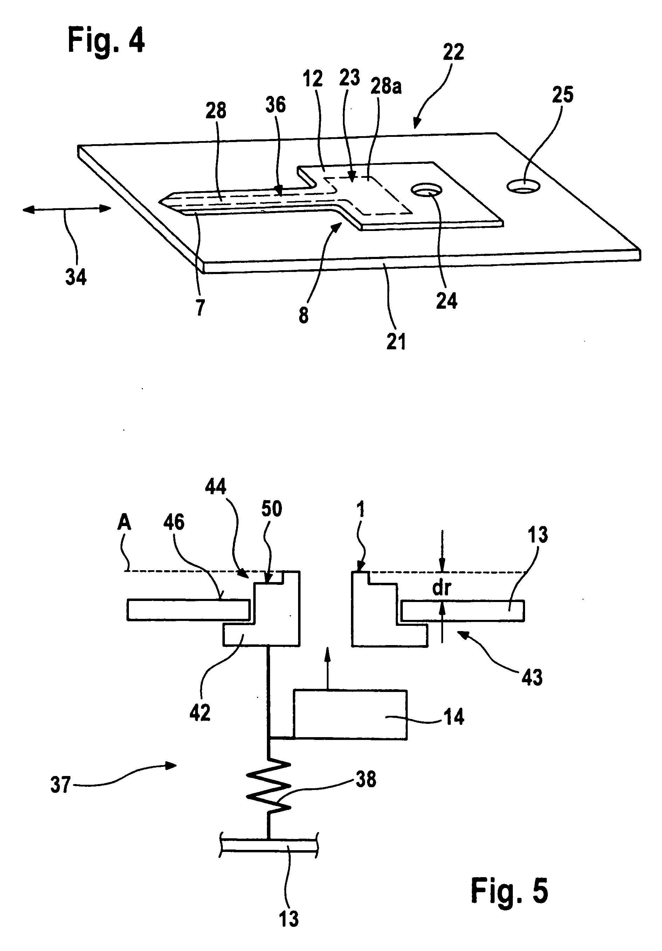

In a preferred embodiment (best shown in Figure 4) lancing element 12 is combined with an analysis element 21, thereby forming an integral lancing and analysis element 22. In this integral element the lancing element 12 is movable, relative to the analysis element 21, in a longitudinal direction symbolized by double arrow 34. An analysis element holder 20 is provided for holding the analysis element 22 inside the instrument 11. In the embodiment shown analysis element holder 20 comprises a coupling recess 25 in analysis element 21 and a corresponding coupling protrusion 27 of the instrument. In a similar manner the lancing element 12 has a coupling recess 24 cooperating with a coupling protrusion 26 of the instrument. These pairs of recesses 24,25 and protrusions 26,27 penetrating into the respective coupling recesses allow the handling of an integral lancing and analysis element 22 which has been inserted into the instrument (Figure 3).

The lancet drive 14 shown in Figure 3 comprises a drive rotor 29 with a cam 30 formed by a groove. Cam 30 and a corresponding cam traveler 31 form a cam drive mechanism which controls a pivoting movement of a drive rod 32 about a pivoting axis 33.

After triggering of a puncture movement (by triggering means not shown) drive rotor 29 turns with high speed (driven by a drive spring also not shown) about its axis 35 and this rotational movement is translated by means of the cam curve formed by groove 30 and traveled by cam traveler 31 into a corresponding pivoting movement of drive rot 32 which again drives a corresponding up and down movement of lancing element 12 to which it is connected by its coupling protrusion 26 penetrating into coupling recess 24. Similar rotor drives for lancing instruments have been described elsewhere. Thus no more detailed description is necessary.

In the preferred embodiment shown in Figure 4 the lancing element 12 is a "direct sampler" having a capillary channel 28 inside its piercing tip 7 and leading up to a sample collection zone 23 of lancing element 12. In sample collection zone 23 capillary channel 28 widens to form a sample reservoir chamber 28a.

During lancing, lancing element 12 performs a puncturing movement by which piercing tip 7 is driven into skin 3. Thereafter, preferably during a retraction phase of the puncturing movement, after the piercing tip 7 has reached its point of maximum displacement (but with the piercing tip still being below the surface of skin 3) sample liquid penetrates - driven by capillary forces - into capillary 28 and reservoir chamber 28a. Thus, in the embodiment shown, capillary 28 and reservoir chamber 28a together form a sample collection device 36 suitable for storing sample liquid, ready for subsequent transfer to an analysis zone 8 of analysis element 21. The volume of sample liquid required for the analysis is determined by the liquid volume taken up by the device, in this case by the combined volume of capillary 28 and reservoir chamber 28a.

Once sample liquid has arrived at sample collection zone 23 it may be transferred to the adjacent analysis zone 8 of analysis element 21 by means of a suitable fluid communication device. Preferably the arrangement is such that in a first configuration no fluid communication between the sample collection zone of lancing element 12 and the analysis element 21 is provided whereas in a second configuration fluid communication takes place. The switching between both configurations can be accomplished by any suitable means, e.g. by pressing zone 23 of lancing element 12 and analysis element 21 together. A more detailed description of such preferred design of an integrated lancing and analysis element with switchable sample transfer is not necessary because it has been described elsewhere.

Of course the invention can also be used with integral lancing and analysis elements having a lancing part and a analysis part fixed to each other. Evidently with such an embodiment no separate holding devices for the two parts are required. Rather only one holding device is provided which simultaneously serves as lancing element holding device and as analysis element holding device.

While preferred devices for holding and moving a lancing element and an analysis element (or an integral lancing and analysis element) in the instrument have been described, many variants are possible. These include a design in which analysis elements and/or lancing elements are fixed to and transported by means of a tape during at least a part of the system operation.

A special feature of the system of the invention relates to a pressing force control device 37 provided in the instrument 11. In the embodiment shown the pressing force control device 37 comprises a spring 38 which is embodied and arranged in such a manner that one end thereof acts against pressure ring 1 and the other end acts against the housing 13. "Acting" in this context does not require immediate contact. Rather it means that the spring exerts a force on the pressure ring and that the corresponding counter-force is (directly or indirectly) borne by the housing.

In the instrument shown in Figure 3 one end of spring 38 rests on a wall of housing 13 and its other end presses against a frame element 39 carrying lancing drive 14. The force of spring 38 is further transmitted from the frame element 39 to pressure ring 1 via pillar elements 40. Pressure ring 1 is embodied as part of a pressure piece 42 which is borne by a pressure ring bearing 43 of housing 13 such that it is axially movable against the force of spring 38.

When a user presses her or his finger tip 2 in the direction of arrow F onto pressure piece 42 with pressure ring 1, the latter moves downwardly against the force of spring 38 (or other spring device). As soon as the contact between pressure piece 42 and housing 13 at pressure ring bearing 43 is interrupted, the force of spring 38 is balanced by the pressing-down force of the finger. In other words the force by which the pressure ring 1 is pressed against the skin is in this status controlled by the pressing force control device 37, embodies here by means spring 38.

The principles used in this design are more clearly apparent from figure 5 showing that spring 38 acts between housing 13 and pressure piece 42 with pressure ring 1. In the specific embodiment shown drive 14 is connected in a defined spatial configuration with pressure ring 1, in such a manner that the distance between the point of maximum displacement of the lancet movement and the pressure ring 1 is independent from the compression status of the spring 38 and the corresponding axial movement of pressure piece 42. Preferably the spatial configuration and hence the distance of the pressure ring from the point of maximum displacement can be varied (between puncturing movements) to set the lancing penetration depth. It is, however, fixed during the interaction of the user with the device, i.e. from the point of time at which the pressure ring is first pressed down until the body part is removed therefrom.

As is well known the force of an elastic spring 38 increases linearly with its elongation (i.e. compression in the case of a compression spring as shown). In the context of the invention the force by which the pressure ring 1 is pressed against the skin shall be controlled closely, i.e. the variation thereof should not exceed the preferred limiting values given above. In order to achieve this end, spring 38 is preferably embodied and arranged in such a manner that it is pre-tensioned. This means that the spring is already compressed (or in the case of an extension spring extended) even if no pressing force is exerted onto pressure ring 1, i.e. pressure ring 1 is in its "home" position resting on the surrounding wall (bearing 43) of housing 13. The degree of this pre-tensioning is such that the force of spring 38 acting on the pressure ring 1 varies by no more than 20 %, preferably no more than 10 % within the spring-loaded movement range of the pressure ring 1.

In the embodiment shown the pressing force control device is adapted to control not only a minimum pressing force but also a maximum pressing force. This is generally favorable but not mandatory. Preferably the maximum pressing force at the time of triggering is not more than 8 N, preferably not more than 7 N.

If a maximum pressing force is to be maintained it is important to make sure, that in the entire movement range the pressing-force acting between the finger 2 (or other body part) and the pressure ring 1 is controlled only by the force of spring 38 balanced by the pressing-downforce of finger 2. This condition would not be met if the movement of pressure ring 1 was influenced or limited by some kind of abutting member or obstacle acting - within its possible movement range - on ring 1. In order to meet this condition a pressure ring movement limiting arrangement 44 (figure 5) is provided by which the maximum displacement of the pressure ring 1 possible by pressing with a finger 2 or other body part is limited within a fully spring-loaded movement range of the pressure ring.

In the preferred embodiment shown in figure 5 this is achieved by a contact surface 46 which is arranged in the vicinity (at the radially outer side) of the pressure ring 1 in such a manner that a body part pressed against the pressure ring 1, and thereby moving the pressure ring, abuts against the contact surface 46. Due to this abutting, the pressure ring can not be moved further, i.e. the possible displacement of the pressure ring (by the body part pressing thereagainst) is limited. With such an embodiment the maximum displacement depends on the distance dr by which the pressure ring protrudes from the instrument housing (contact surface 46). When pressure piece 42 with pressure ring 1 is pressed downwardly this movement is discontinued when finger tip 2 contacts the surface of housing 13 in the vicinity of the pressure ring 1.

In this context it is also favorable if the design is such that the maximum displacement of the pressure ring 1 during practical use is small. Preferably it should be less than 3 mm, more preferably less than 2 mm and even more preferably less than 1 mm. Therefore the distance dr of the plane A of pressure ring 1 and the adjacent housing surface should not be too large. Preferred maximum values can be calculated by adding 0.5 mm to the mentioned maximum displacement values. On the other hand dr should not be too small, among others because it is favorable for the handling of the instrument if a protrusion of pressure ring 1 versus the adjacent housing area simplifies the finding of a suitable finger position for the user. Therefore this protrusion, i.e. the distance dr, should be at least 0.2 mm, and preferably at least 0.5 mm.

Of course the construction part at which the pressure ring is provided can have many different shapes and designs. In contrast to some earlier devices it should be non-deformable in the sense that it should not be visibly deformed during normal use of the system. As stated earlier the term "pressure ring" refers to the ring-shaped surface of the respective part which in practical use, i.e. under the conditions prevailing in using of the particular instrument) bears the major part of the pressing force acting between the instrument and the skin. Of course this ring-shaped contact surface (i.e. the pressure ring) can have varying shapes including e.g. slightly rounded edges. A suitable exact shape and width of the pressure ring can be determined experimentally on the basis of the information contained herein. The ring should protrude from any adjacent housing surface by a sufficient distance to allow easy tactile recognition thereof by the user.

Furthermore the term "pressure ring" does not have to be understood as being limited to an uninterrupted ring. Rather the ring shaped surface contacting the skin can have interruptions (for example by recesses) which should, however, be small enough not to spoil the described function of the pressure ring.

In the preferred embodiment shown in Figure 3 the instrument furthermore comprises a pressure-ring-movement detection device 45. In the context of the second main aspect of the invention it is preferably a part of a MITP control device. Means for movement detection are well known, for example a light barrier 46 as shown symbolically in the drawing. Such a device detects the movement of pressure piece 42, and hence of pressure ring 1, upon pressing down from its "home" position by finger 2. Such detection allows several favorable functions including an indication to the user (via display 16 or generators of audible or tactile signals 18 and 19) that the instrument is "ready for lancing". Alternatively or additionally the signal of the pressure-ring-movement detection device can be used for automatically triggering the lancing movement, possibly after a delay time as described above.

Figure 3 furthermore shows an analysis measurement device 47 as part of the instrument 11. This can be any device which is suitable to measure a value of a measurement quantity relating to a change of analysis element 21, which change is a measure of the desired analytical value. In the case shown, the analysis measurement device is embodied for a photometric measurement of a detection area in the analysis zone 8 of analysis element 21 including a light source 48, a light detector 49 and corresponding light guide means symbolized by a lens 50. Other types of analysis measurement devices could be used as well, in particular electrical measurement devices, as are common for the evaluation of electrochemical analysis elements.

In the system shown in Figures 2 to 5 a MITP control device 17 in accordance with the second main aspect of the invention makes use of the pressure-ring-movement detection device 45. Once MITP control device 17 signals the start of an MITP it generates a signal by at least one of signal generators 18 and 19 and/or automatically triggers a puncturing movement of lancet drive 14. The end of the MITP period is determined by the measurement and evaluation electronics 15, for example on the basis of a predefined time period required for generation and transfer of sufficient amount of sample liquid from the finger tip 2. Alternatively the status of a sufficient sample transfer may be separately detected by suitable sample transfer detection means as are known in the art, e.g. photometric detection of the sample transported in the integral lancing and analysis element 22 or by electrical contacts detecting that sample liquid transported therein has reached a certain point in its transport path. Preferably - as in the embodiment shown - the application force control device makes sure that during the entire MITP the pressing force is within the range given by the above identified limiting values. Preferably the variation of the pressing force during the MITP should be within the above identified variation limitations which are much smaller.

## Claims

1. Instrument for producing a sample of body liquid for analysis by piercing the skin with a lancing element (12) having a skin piercing tip (7)
said instrument (11) having
- a housing (13)
- a lancing drive (14) within said housing (13) for being connected to a lancing element (12) and for driving a lancing element (12) connected thereto in a puncturing movement in which the lancing element (12) moves, after triggering the puncturing movement, in a puncture direction until it reaches a point (P) of maximum displacement and in a reverse direction after it has reached the point (P) of maximum displacement,
- a pressure ring (1) surrounding a skin contact opening (4) and being adapted for pressing against the skin,
wherein
the skin contact opening (4) has an opening area corresponding to a circle with a diameter of at least 1.5 mm and not more than 4 mm, and
the instrument comprises a pressing force control device (37) for controlling the pressing force between the pressure ring (1) and the skin (3) at the time of triggering the puncturing movement, to be at least 3 N, preferably at least 4 N, most preferably at least 5 N.

2. Instrument according to claim 1, wherein the opening area of the skin contact opening (4) corresponds to a circle with a diameter of at least 2 mm, preferably at least 2.5 mm.

3. Instrument according to any one of the preceding claims
wherein
the pressure ring (1) has on at least a part of its circumference a width (b) of not more than 1.5 mm, preferably not more than 1.2 mm and most preferably not more than 1 mm.

4. Instrument according to claim 3, comprising a peripheral surface portion (50) surrounding the pressure ring and recessed from the pressure ring such that the pressure ring protrudes in the puncturing direction from the peripheral surface portion.

5. Instrument according to claim 4, wherein the pressure ring (1) protrudes from the peripheral surface by a distance (h) of at least 0.5 mm, preferably by at least 0.8 mm.

6. Instrument according to any one of the preceding claims,
wherein
the instrument comprises a pressing force control device (37) for controlling the pressing force between the pressure ring (1) and the skin (3) at the time of triggering the puncturing movement, to be at least 3 N, and
the lancing drive (14) and the pressure ring (1) are so positioned relative to each other that a projecting distance (s) by which the skin piercing tip (7) of the lancing element (12) extends out of the pressure ring plane, when the lancing element (12) is in the point of maximum displacement, is not more than 0.3 mm, preferably not more than 0.2 mm and most preferably not more than 0.1 mm.

7. Instrument according to any one of claims 3 to 6, wherein the skin contact opening (4) has an opening area corresponding to a circle with a diameter of at least 1.5 mm, preferably at least 2 mm and, most preferably at least 2.5 mm and not more than 8 mm, preferably not more than 5 mm, most preferably not more than 3.5 mm.

8. Instrument according to any one of claims 3 to 7, wherein the pressing force control device (37) is adapted for controlling the pressing force between the pressure ring (1) and the skin (3) at the time of triggering the puncturing movement, to be at least 4 N, preferably at least 5 N.

9. Instrument according to any one of the preceding claims, wherein the pressure ring (1) is non-deformable.

10. Instrument according to any one of the preceding claims comprising
a sample collection device (36) for collecting an amount of sample liquid required for analysis and
a holding device (20) for holding an analysis element in said housing such that a sample of body liquid is transported thereto from the sample collection device (36) for analysis.

11. System for producing a sample of body liquid for analysis by piercing the skin comprising a reusable hand-held-instrument (11) according to any one of the preceding claims and a lancing element (12) which is adapted to be interchangeably connected to the drive (14) of the instrument.

12. System according to claim 11, wherein the lancing element (12) is a part of an integral lancing- and analysis element (22).

13. System according to any one of claims 11 or 12, wherein the lancing element (12) is a direct sampler having a capillary channel (28) for transporting sample liquid produced by piercing the skin by means of capillary flow to a sample collection zone (23) thereof.

14. System according to any one of claims 11 to 13, wherein the sample volume required for the analysis is not more than 300 nl, preferably not more than 200 nl and most preferably not more than 100 nl.

## Patentansprüche

1. Instrument zur Erzeugung einer Körperflüssigkeitsprobe für Analysezwecke durch Einstechen in die Haut mittels eines Stechelementes (12), das eine Hautstechspitze (7) hat,
wobei das Instrument (11) aufweist
- ein Gehäuse (13)
- einen Stechantrieb (14) in dem Gehäuse (13), der dazu ausgebildet ist, mit einem Stechelement (12) verbunden zu werden und ein mit ihm verbundenes Stechelement (12) bei einer Stechbewegung anzutreiben, bei der das Stechelement (12) nach dem Auslösen der Stechbewegung in eine Einstichrichtung bewegt wird, bis es einen Punkt (P) seiner maximalen Auslenkung erreicht und in eine Rückwärtsrichtung, nach Erreichen des Punktes (P) der maximalen Auslenkung, bewegt wird,
- einen Druckring (1), der eine Hautkontaktöffnung (4) umgibt und dazu ausgebildet ist, gegen die Haut zu drücken,
wobei
die Hautkontaktöffnung (4) einen Öffnungsquerschnitt aufweist, der einem Kreis mit einem Durchmesser von mindestens 1,5 mm und höchstens 4 mm entspricht, und
das Instrument eine Druckkraft-Kontrolleinrichtung (37) aufweist, um die Druckkraft zwischen dem Druckring (1) und der Haut (3) zum Zeitpunkt des Auslösens der Stechbewegung so zu kontrollieren, dass sie mindestens 3 N, vorzugsweise mindestens 4 N, besonders bevorzugt mindestens 5 N beträgt.

2. Instrument nach Anspruch 1, bei welchem der Öffnungsquerschnitt der Hautkontaktöffnung (4) einem Kreis mit einem Durchmesser von mindestens 2 mm, vorzugsweise mindestens 2,5 mm entspricht.

3. Instrument nach einem der vorhergehenden Ansprüche, bei welchem der Druckring (1) auf mindestens einem Teil seines Umfangs eine Breite (b) von höchstens 1,5 mm, vorzugsweise höchstens 1,2 mm und besonders bevorzugt höchstens 1 mm hat.

4. Instrument nach Anspruch 3, welches einen peripheren Oberflächenbereich (50) aufweist, der den Druckring umgibt und gegenüber dem Druckring derartig zurückspringt, dass der Druckring in Einstichrichtung gegenüber dem peripheren Oberflächenbereich vorsteht.

5. Instrument nach Anspruch 4, bei welchem der Druckring (1) gegenüber dem peripheren Oberflächenbereich um einen Abstand (h) von mindestens 0,5 mm, vorzugsweise mindestens 0,8 mm vorsteht.

6. Instrument nach einem der vorhergehenden Ansprüche,
wobei
das Instrument eine Druckkraft-Kontrolleinrichtung (37) aufweist, um die Druckkraft zwischen dem Druckring (1) und der Haut (3) zum Zeitpunkt des Auslösens der Stechbewegung so zu kontrollieren, dass sie mindestens 3 N beträgt, und
der Stechantrieb (14) und der Druckring (1) relativ zueinander so angeordnet sind, dass ein Vorspring-Abstand (s), um den die Hautstechspitze (7) des Stechelementes (12) aus der Ebene des Druckrings vorspringt, wenn sich das Stechelement (12) am Punkt seiner maximalen Auslenkung befindet, höchstens 0,3 mm, vorzugsweise höchstens 0,2 mm und besonders bevorzugt höchstens 0,1 mm beträgt.

7. Instrument nach einem der Ansprüche 3 bis 6, bei welchem die Hautkontaktöffnung (4) einen Öffnungsquerschnitt hat, der einem Kreis mit einem Durchmesser von mindestens 1,5 mm, vorzugsweise mindestens 2 mm, besonders bevorzugt mindestens 2,5 mm und höchstens 8 mm, vorzugsweise höchstens 5 mm, besonders bevorzugt höchstens 3,5 mm entspricht.

8. Instrument nach einem der Ansprüche 3 bis 7, bei welchem die Druckkraft-Kontrolleinrichtung (37) dazu ausgebildet ist, die Druckkraft zwischen dem Druckring (1) und der Haut (3) zum Zeitpunkt des Auslösens der Stechbewegung so zu kontrollieren, dass sie mindestens 4 N, vorzugsweise mindestens 5 N beträgt.

9. Instrument nach einem der vorhergehenden Ansprüche, bei welchem der Druckring (1) nicht-deformierbar ist.

10. Instrument nach einem der vorhergehenden Ansprüche, umfassend
eine Probensammeleinrichtung (36) zum Sammeln einer für eine Analyse benötigten Menge an Probenflüssigkeit und
eine Halteeinrichtung (20) zum Halten eines Analyseelementes in dem Gehäuse dergestalt, dass eine Probekörperflüssigkeit von der Probensammeleinrichtung (36) zur Analyse zu dem Analyseelement transportiert wird.

11. System zur Erzeugung einer Körperflüssigkeitsprobe für Analysezwecke durch Einstechen in die Haut, umfassend ein wiederverwendbares Handinstrument (11) nach einem der vorhergehenden Ansprüche und ein Stechelement (12), welches dazu ausgebildet ist, auswechselbar mit dem Stechantrieb (14) des Instruments verbunden zu werden.

12. System nach Anspruch 11, bei welchem das Stechelement (12) ein Teil eines integralen Stech- und Analyseelementes (22) ist.

13. System nach einem der Ansprüche 11 oder 12, bei welchem das Stechelement (12) ein Direktsammler ist, der einen Kapillarkanal (28) aufweist, um mittels Kapillarströmung Probenflüssigkeit, die durch einen Stich in die Haut gewonnen wurde, zu einer Probensammelzone (23) des Direktsammlers zu transportieren.

14. System nach einem der Ansprüche 11 bis 13, bei welchem das Probenvolumen, welches für die Analyse benötigt wird, höchstens 300 nl, vorzugsweise höchstens 200 nl und besonders bevorzugt höchstens 100 nl beträgt.

## Revendications

1. Instrument de production d'un échantillon de liquide corporel pour analyse par perçage de la peau avec un élément de piquage (12) ayant une pointe (7) de perçage de la peau
ledit instrument (11) ayant
- un logement (13)
- un entraîneur (14) de piquage à l'intérieur dudit logement (13) destiné à être raccordé à un élément de piquage (12) et à entraîner un élément de piquage (12) raccordé à celui-ci dans un mouvement de piquage dans lequel l'élément de piquage (12) se déplace, après déclenchement du mouvement de piquage, dans un sens de piquage jusqu'à ce qu'il atteigne un point (P) de déplacement maximum et dans un sens inverse après qu'il a atteint le point (P) de déplacement maximum,
- une bague de pression (1) entourant une ouverture (4) de contact avec la peau et étant adaptée à être pressée contre la peau,
dans lequel
l'ouverture (4) de contact avec la peau a une superficie d'ouverture correspondant à un cercle avec un diamètre d'au moins 1,5 mm et pas plus de 4 mm, et
l'instrument comprend un dispositif (37) de contrôle de force de pression pour contrôler la force de pression entre la bague de pression (1) et la peau (3) au moment de déclenchement du mouvement de piquage, pour qu'elle soit d'au moins 3 N, préférablement au moins 4 N, le plus préférablement au moins 5 N.

2. Instrument selon la revendication 1, dans lequel la superficie d'ouverture de l'ouverture (4) de contact avec la peau correspond à un cercle avec un diamètre d'au moins 2 mm, préférablement au moins 2,5 mm.

3. Instrument selon l'une quelconque des revendications précédentes
dans lequel
la bague de pression (1) a, sur au moins une partie de sa circonférence, une largeur (b) de pas plus de 1,5 mm, préférablement pas plus de 1,2 mm et le plus préférablement pas plus de 1 mm.

4. Instrument selon la revendication 3, comprenant une partie de surface périphérique (50) entourant la bague de pression et en renfoncement par rapport à la bague de pression de manière à ce que la bague de pression fasse saillie dans le sens de piquage à partir de la partie de surface périphérique.

5. Instrument selon la revendication 4, dans lequel la bague de pression (1) fait saillie à partir de la partie de surface périphérique d'une distance (h) d'au moins 0,5 mm, préférablement d'au moins 0,8 mm.

6. Instrument selon l'une quelconque des revendications précédentes
dans lequel
l'instrument comprend un dispositif (37) de contrôle de force de pression pour contrôler la force de pression entre la bague de pression (1) et la peau (3) au moment de déclenchement du mouvement de piquage, pour qu'elle soit d'au moins 3 N, et
l'entraînement (14) de piquage et la bague de pression (1) sont positionnés l'un par rapport à l'autre de manière à ce qu'une distance de projection (s) de laquelle la pointe (7) de perçage de la peau de l'élément de piquage (12) s'étend hors du plan de bague de pression, lorsque l'élément de piquage (12) est au point de déplacement maximum, ne soit pas plus de 0,3 mm, préférablement pas plus de 0,2 mm et le plus préférablement pas plus de 0,1 mm.

7. Instrument selon l'une quelconque des revendications 3 à 6, dans lequel l'ouverture (4) de contact avec la peau a une superficie d'ouverture correspondant à un cercle avec un diamètre d'au moins 1,5 mm, préférablement au moins 2 mm et le plus préférablement au moins 2,5 mm et de pas plus de 8 mm, préférablement pas plus de 5 mm, le plus préférablement pas plus de 3,5 mm.

8. Instrument selon l'une quelconque des revendications 3 à 7, dans lequel le dispositif (37) de contrôle de force de pression est adapté à contrôler la force de pression entre la bague de pression (1) et la peau (3) au moment de déclenchement du mouvement de piquage pour qu'elle soit d'au moins 4 N, préférablement au moins 5 N.

9. Instrument selon l'une quelconque des revendications précédentes, dans lequel la bague de pression (1) est non déformable.

10. Instrument selon l'une quelconque des revendications précédentes comprenant
un dispositif (36) de collecte d'échantillon pour collecter une quantité d'échantillon de liquide requise pour analyse et
un dispositif de maintien (20) pour maintenir un élément d'analyse dans ledit logement de manière à ce qu'un échantillon de liquide corporel soit transporté jusqu'à celui-ci à partir du dispositif (36) de collecte d'échantillon pour analyse.

11. Système de production d'un échantillon de liquide corporel par perçage de la peau comprenant un instrument (11) à main réutilisable selon l'une quelconque des revendications précédentes et un élément de piquage (12) qui est adapté à être raccordé de façon interchangeable à l'entraîneur (14) de l'instrument.

12. Système selon la revendication 11, dans lequel l'élément de piquage (12) est une partie d'un élément intégré de piquage et d'analyse (22).

13. Système selon l'une quelconque des revendications 11 ou 12, dans lequel l'élément de piquage (12) est un échantillonneur direct ayant un canal capillaire (28) pour transporter un échantillon de liquide produit par perçage de la peau au moyen d'un écoulement capillaire jusqu'à une zone (23) de collecte d'échantillon de celui-ci.

14. Système selon l'une quelconque des revendications 11 à 13, dans lequel le volume d'échantillon requis pour l'analyse n'est pas de plus de 300 nl, préférablement pas plus de 200 nl et le plus préférablement pas plus de 100 nl.
